# EUROPEAN PATENT SPECIFICATION

(11) **EP 1 830 805 B1**
(45) Date of publication and mention of the grant of the patent: **25.06.2014**
(21) Application number: 05849698.5
(22) Date of filing: 30.11.2005
(51) Int. Cl.: A61K 8/27, A61K 9/00, A61K 6/00, A61K 31/74, A61Q 17/04, A61Q 19/00, A61K 8/21, A61K 8/06

(54) **COMPOSITIONS COMPRISING POLYMERIC EMULSIFIERS AND METHODS OF USING THE SAME**
ZUSAMMENSETZUNGEN AUS POLYMER-EMULGATOREN UND ANWENDUNGSVERFAHREN
COMPOSITIONS COMPRENANT DES EMULSIFIANTS POLYMERIQUES ET PROCEDES D'UTILISATION CORRESPONDANTS

(30) Priority: 17.12.2004 US 16158
(43) Date of publication of application: 12.09.2007
(73) Proprietor: JOHNSON & JOHNSON CONSUMER COMPANIES, INC., Skillman, NJ 08558 (US)
(72) Inventor: COSSA, Anthony, J., Branchburg, NJ 08876 (US); NIKOLOVSKI, Janeta, Princeton, NJ 08542 (US); LIBRIZZI, Joseph, Hillsborough, NJ 08844 (US); WIEGAND, Benjamin, Carl, Yardley, PA 19067 (US)
(74) Representative: Kirsch, Susan Edith
(86) International application number: PCT/US2005/043174
(87) International publication number: WO 2006/065529

(56) References cited:
- EP-A1- 0 995 433
- EP-A1- 1 203 789
- WO-A1-03/088965
- DE-A1- 10 162 058
- US-A- 6 013 269
- US-B1- 6 635 702
- US-B2- 6 696 068

## Description

### FIELD OF THE INVENTION

The present invention relates to compositions comprising polymeric emulsifiers and, more particularly, to emulsion compositions comprising polymeric emulsifiers which exhibit unexpected rinse-resistance properties.

### BACKGROUND

Conventional oil-in-water emulsions include monomeric surfactant emulsifiers to stabilize oil droplets in an external water phase. Formulations developed using this approach typically possess excellent aesthetics, but are not capable of providing a suitably durable, water-resistant barrier when applied to a substrate such as, for example, the skin. Additionally, applicants have recognized that monomeric surfactant emulsifiers used to stabilize the emulsion tend to suffer from such drawbacks as contributing to irritation of the skin and eyes. Furthermore, applicants have recognized that excess amounts of surfactant emulsifiers may also cause stickiness or foaming upon application to skin, which may be undesirable for certain products, such as moisturizers, lotions, and the like.

One method of improving the water-resistant barrier formed from an emulsion composition is to invert the emulsion, i.e., to use an oil exterior phase, a so-called "water-in-oil" emulsion system. While these systems tend to improve the barrier properties, they tend to do so at the cost of aesthetics, as they are typically perceived as very greasy and suffer from poor spreadability. Additionally, they often still rely on monomeric surfactant emulsifiers to stabilize the emulsion and therefore suffer from similar problems attributed by applicants to monomeric surfactant emulsifiers in oil-in-water emulsion systems.

Recently, polymers have been developed which can stabilize oil droplets in a water-based system without the use of surfactant emulsifiers. U.S. Pat. No. 5,373,044 (Adams et al.) describes an interpolymer of at least one olefinically unsaturated carboxylic acid containing at least one activated carbon-to-carbon olefinic double bond and at least one carboxyl group which has both thickening and emulsifying/dispersing properties. Related commercially available products include Pemulen® TR-1 and TR-2 from Noveon, Inc. Yet another example of a polymer that possesses emulsifying/dispersing properties can be found in U.S. Pat. No. 6,489,395 (Loffler) and U.S. Pat. No. 6,682,750 (Loffler et al.) which describe crosslinked water-soluble or water-swellable copolymers based on acrylamidoalkylsulfonic acids and cyclic N-vinylcarboxamides and/or linear N-vinylcarboxamides. Related commercially available products include Aristoflex® AVC and Aristoflex® HMB from Clariant Corporation. Other commercially available polymeric emulsifiers include a homopolymer of acrylamidoalkyl sulfonic acid, commercially available as Granthix APP from Grant Industries, Inc.

The use of polymeric emulsifiers in place of monomeric surfactants has resulted in the ability to form stable oil-in-water emulsions that can be formulated to be free of surfactant, possess favorable aesthetics and leave a water-resistant barrier. One method for producing such lotions utilizing a copolymer of acrylic acid and long chain acrylate as the polymeric emulsifier is described in U.S. Pat. No. 5,004,598 (Lockhead et al.).

Applicants have recognized, however, that such conventional emulsion compositions comprising polymeric emulsifiers nevertheless have several disadvantages associated therewith. For example, one disadvantage associated with such emulsion compositions is that, when applied to the skin, the compositions tend to demonstrate problems wetting the surfaces to which they are applied. Such relatively poor wetting properties result in poor consumer acceptability in many applications (e.g., poor diaper rash or sunscreen performance, poor make-up coverage, poor aesthetics, and the like).

To overcome such disadvantages, applicants have recognized that it would be advantageous to incorporate a wetting agent into the polymeric emulsifier emulsion to enhance the spreading of the formula on skin and for compositions including hydrophilic particulates, to facilitate the incorporation of hydrophilic particulate into the oil film. However, incorporating a wetting agent into conventional emulsion compositions typically results in one or more undesirable situations such as the oil is re-emulsified (preventing optimal formation of the oil film, thus reducing water-resistance imparted) or adversely impact the aesthetics and irritation of the formula.

Another disadvantage associated with conventional emulsion compositions comprising polymeric emulsifiers is that when these compositions include hydrophilic particulates (i.e., oxides and the like) that are intended to be incorporated into the hydrophobic film formed via application of the emulsion composition to a surface, such as when formulating a diaper rash cream, the oil film cannot wet the pigment and the pigment is therefore readily washed away upon urination. To counter this problem, it is possible to coat the hydrophilic particle with a hydrophobic coating. Unfortunately, coating the particle also suffers from drawbacks, such as by adding more raw material expense, making phase stability of the composition more difficult, and in some cases, preventing the particulate from imparting a chemical function (e.g., preventing hydrophilic and partially soluble ZnO from mitigating diaper rash).

Accordingly, applicants have recognized the need for emulsion compositions that overcome, in whole or in part, the aforementioned disadvantages.

US 6,635,702 discloses an aqueous composition containing a cross-linked alkaliswellable acrylate copolymer rheology modifier, a surfactant, an alkaline material, a silicone and a pearlescent material. The composition can be used as a pearlized conditioning shampoo.

US 6,696,068 discloses a cosmetic cream cleansing composition based on the presence of a silicate, a cross-linked carboxyvinyl polymer, a silicone polyol sulfosuccinate and a carrier. The composition can contain a surfactant.

EP 1 203 789 Al discloses a composition for use as a tightening agent in the cosmetic field to smooth skin. The composition can contain a mixed silicate, a polysaccharide, a cross-linked poly(2-acylamido-2-methylpropane sulfonic acid) polymer which is at least 90% neutralized, an emulsifier selected from the group consisting of polyoxyethylenated and/or polyoxypylenated fatty alcohol ethers, alkyl ether sulfates, sulfosuccinates, isethionates and mixtures thereof.

EP 0 995 433 Al discloses a retinol stabilised cleansing composition that contains a skin enhancing agent such as retinol, glycerine, one or more emollients and a salt of cocoyl isethionate, a monoalkyl phosphate or a salt thereof, or a combination thereof, neutralised to a pH ranging from 5 to 8.

### SUMMARY OF THE INVENTION

According to one aspect of the present invention, there is provided a composition as set out in claim 1. Preferred features of the composition are set out in dependent claims 2 to 9.

According to another aspect of the present invention, there is provided the personal care composition of the present invention for use in a method of mitigating diaper dermatitis comprising applying the composition to skin, e.g. human skin, in need of diaper dermatitis mitigation, as set out in claim 10.

### DESCRIPTION OF PREFERRED EMBODIMENTS

Applicants have discovered unexpectedly that polymeric emulsifiers, hydrophobic agents, and certain wetting agents can be combined with hydrophilic particulate matter to form compositions that overcome one or more of the disadvantages associated with conventional emulsion compositions. That is, applicants have recognized that the emulsion compositions of the present invention tend to exhibit unexpectedly high rinse resistance and particulate deposition properties, as well as relatively high stability, low irritation, and/or high wetting as compared to conventional emulsion compositions.

In particular, applicants have tested the rinse-resistance and deposition properties associated with compositions of the present invention via the "Skin Whitening Test", described in detail below, which measures the Delta L value of a particular composition (wherein a higher Delta L value represents a composition having associated therewith the desirable properties of higher deposition of particulate matter and higher rinse-resistance). Applicants have discovered unexpectedly that the compositions of the present invention exhibit a Delta L value that is unexpectedly high as compared to conventional compositions. For example, in certain embodiments, the present compositions exhibit a Delta L value that is at least about 2.5 or higher, preferably at least about 3 or higher, more preferably at least about 3.5 or higher, more preferably at least about 4 or higher, more preferably at least about 4.5 or higher and even more preferably at least about 5 or higher. In certain preferred embodiments, such values tend to be at least about 5, preferably about 6-10 or more, times greater than comparable compositions outside of the claimed invention. Applicants have recognized that such significant difference is not only unexpected, but may also be used to significant advantage in a variety of applications.

As used herein, the term polymeric emulsifier" refers generally to a polymer having both hydrophilic and hydrophobic moieties that is capable of contributing to the formation of a stable emulsion between an oil phase and an aqueous phase. Any of a variety of suitable polymeric emulsifiers may be used according to the present invention. In certain preferred embodiments, applicants have recognized it is desirable to use one or more polymeric emulsifiers which tends to provide shelf stability to the composition into which it is added and/or tends to facilitate the deposition of hydrophobic agent onto a substrate. In addition, certain preferred polymeric emulsifiers comprise those compounds that are water-soluble and are capable of forming a phase stable emulsion, preferably stable for at least about 1 week, more preferably at least about a month, of a hydrophobic agent of the present invention in water. (As used herein a material is defined as "water-soluble", if it is possible to form a clear solution by adding only 0.5% by weight of the material in deionized water that is stable at room temperature (no settling or phase-instability) for 48 hours.) Certain preferred polymeric emulsifiers of the present invention are salt-sensitive, in that, their solubility in water is reduced, often dramatically, in the presence of electrolytes (such as electrolytes typically present on the surface of skin). A polymeric emulsifier is defined as "salt-sensitive" if it loses its ability to remain phase stable in aqueous solution when sodium chloride has been added. Specifically, a "salt sensitive" polymeric emulsifier will show phase separation and/or a 30% or more change in viscosity (measured using a Brookfield viscometer with an LVT2 spindle at 12 RPM) if 3% sodium chloride is added to a homogenous solution of 1% (active) polymeric emulsifier in deionized water.

The polymers for use as polymeric emulsifiers in the present invention may be of any suitable molecular weight. In certain embodiments of the invention, the polymeric emulsifier has a weight average molecular weight that is preferably greater than about 500,000, more preferably greater than about 250,000, and even more preferably greater than about 100,000.

Polymeric emulsifiers suitable for the present invention may comprise an associative polymer, i.e., a polymer formed from monomers such that individual repeat units are hydrophilic, such as may be formed by addition polymerization of such as acids as acrylic, methacrylic, maleic, itaconic, and the like or combinations to form copolymers thereof.

Notable commercially available polymeric emulsifiers include, but are not limited to, salt sensitive, hydrophobically modified polyacrylic acid commercially under the tradename Pemulen® TR-1 and TR-2 by Noveon, Inc., water-soluble or water-swellable copolymers based on acrylamidoalkyl sulfonic acid and cyclic N-vinylcarboxamides commercially available under the tradename Aristoflex® AVC by Clariant Corporation; water-soluble or water-swellable copolymers based on acrylamidoalkyl sulfonic acid and hydrophobically modified methacrylic acid commercially available under the tradename Aristoflex® HMB by Clariant Corporation; and a homopolymer of acrylamidoalkyl sulfonic acid commercially available under the tradename Granthix APP by Grant Industries, Inc.

Another class of notable polymeric emulsifier includes hydrophobically-modified, crosslinked, anionic acrylic copolymers, including random polymers, but may also exist in other forms such as block, star, graft, and the like. In one embodiment, the hydrophobically modified, crosslinked, anionic acrylic copolymer may be synthesized from at least one acidic monomer and at least one hydrophobic ethylenically unsaturated monomer. Examples of suitable acidic monomers include those ethylenically unsaturated acid monomers that may be neutralized by a base. Examples of suitable hydrophobic ethylenically unsaturated monomers include those that contain a hydrophobic chain having a carbon chain length of at least about 3 carbon atoms.

Other materials that may be suitable polymeric emulsifiers include ethylene oxide/ propylene oxide block copolymers, sold under the trade name PLURONIC, available from BASF corporation of Parsippany, N.J., modified cellulose polymers such as those modified cellulose polymers described by the trade name KLUCEL, available from Hercules Corporation of Wilmington, DE.

In certain preferred embodiments of the invention, the compositions include a salt sensitive polymeric emulsifier selected from a group consisting of a salt sensitive, hydrophobically modified polyacrylic acid such as Pemulen® TR-1 and TR-2 by Noveon, Inc, an acrylamidoalkyl sulfonic acid, cyclic N-vinylcarboxamides; water-soluble or water-swellable copolymers based on acrylamidoalkyl sulfonic acid and cyclic N-vinylcarboxamides commercially available under the tradename Aristoffex® AVC by Clariant Corporation; water-soluble or water-swellable copolymers based on acrylamidoalkyl sulfonic acid and hydrophobically modified methacrylic acid commercially available under the tradename Aristoflex® HMB by Clariant Corporation and a homopolymer of acrylamidoalkyl sulfonic acid commercially available under the tradename Granthix APP by Grant Industries, Inc.; a hydrophobic agent such as an emollient; e.g., mineral oil, petrolatum, or silicone oil; a wetting agent selected from the group consisting of sulfosuccinates and isethionates; and a hydrophilic particle as an oxide, especially zinc oxide or titanium oxide. In an especially preferred embodiment, the composition meets the limitations specified above and also is substantially free of monomeric surfactant emulsifiers.

Any suitable amounts of polymeric emulsifier may be used in the compositions of the present invention. In certain preferred embodiments, the compositions of the present invention comprise at least about 0.3 weight percent, for example, between about 0.3% and about 3%, between about 0.3% and about 2%, and between about 0.3% and about 1%. As used herein and throughout the application, all percents represent percent by weight of active based on the total weight of composition, unless otherwise indicated.

Any of a variety of hydrophobic materials that are water-insoluble, liquid at room temperature, and capable of forming a water-resistant barrier film or coating on a substrate when used in a composition of the present invention, are suitable for use herein as a hydrophobic agent. (As used herein, the term "water-insoluble" refers to a material that when added to deionized water to a concentration by weight of 0.5% (with no other additives) at room temperature, cannot be made to form a clear homogeneous mixture for a period of time lasting at least 48 hours.)

Examples of suitable hydrophobic agents include, but are not limited to materials such as mineral oils, petrolatum, vegetable oils (glyceryl esters of fatty acids, triglycerides), waxes and other mixtures of esters, not necessarily esters of glycerol; polyethylene and non-hydrocarbon based oils such as dimethicone, silicone oils, silicone gums, and the like. In certain embodiments, preferred hydrophobic agents include emollients such as mineral oil, silicone oils, pertrolatum, combinations thereof, and the like.

Any suitable amounts of hydrophobic agent may be used in the compositions of the present invention. In certain embodiments, the hydrophobic agent is present in the composition in a concentration that is at least about 0.1 weight percent, such as from about 0.1% to about 50%, preferably from about 0.1% to about 40%, and even more preferably from about 0.1% to about 30%.

In certain preferred embodiments, the polymeric emulsifier and the hydrophobic agent are present in a respective weight ratio from about 60:1 1 to about 1:150, preferably from about 40:1 to about 1:120, and more preferably from about 20:1 to about 1:100.

The wetting agent facilitates the wetting of a substrate with a composition of the present invention and allows the hydrophobic agent of the composition to tend to remain on the substrate (for example by avoiding re-emulsification of the hydrophobic agent).

Suitable wetting agents are, disodium laureth sulfosuccinate, dioctyl sodium sulfosuccinate, sodium methyl 2-sulfolaurate, sodium cocoyl isethionate, and sodium lauryl sulfoacetate. A variety of such wetting agents are available commercially from various sources including McIntyre Group Limited (disodium laureth sulfosuccinate sold under the tradename MACKANATE EL, dioctyl sodium sulfosuccinate sold under the tradename MACKANATE DOS 70), Stepan Company (sodium methyl 2-sulfolaurate sold under the tradename ALPHA STEP PC 48, sodium lauryl sulfoacetate sold under the tradename LANTHANOL LAL), and Clariant Corporation (sodium cocoyl isethionate sold under the tradename HOSTAPON SCI 85).

The present compositions comprise at least 0.01 weight percent of wetting agent to 5%, preferably from about 0.01 % to about 3%, and more preferably from about 0.1% to about 2%.

As used herein, the term "hydrophilic particulate" refers generally to any oxide particle (substantially spherical, porous, or high aspect ratio) that tends to be solid at room temperature, is generally hydrophilic in nature, and tends to be either insoluble, sparingly soluble, and/or dissolves in water only at a very slow rate. Any such suitable hydrophilic particulate may be used according to the present invention. Applicants have recognized that for certain embodiments, the hydrophilic particulate is preferably selected to be dispersible in water and have a particle diameter of from about 0.01 µm to about 500 µm preferably from about 0.1 µm to 500 about µm, more preferably from about 0.5 µm to about 400 µm, more preferably from 1.0 µm to about 300 µm, and more preferably from about 10 µm to about 250 µm. In certain embodiments, the hydrophilic particulate is essentially free of one or more coatings comprising one or more hydrophobic moieties (e.g., coatings of fatty acids, fatty esters, hydrophobic surface modification such as from certain organosilanes, and the like) that tend to cancel the hydrophilic nature of the particulate. Examples of hydrophilic particles suitable for use herein include metal oxides such as zinc oxide, titanium dioxide, transition metal oxides or other oxide pigments, combinations of two or more thereof, and the like. For certain preferred embodiments, zinc oxide is particularly notable.

Any suitable amount of hydrophilic particulate may be used in the compositions of the present invention. In certain embodiments, the present compositions comprise from about 0.01 percent to about 50 percent, preferably from about 0.1 percent to about 45 percent, and more preferably, from about 0.1 percent to about 40 percent of a hydrophilic particle.

In certain preferred embodiments, the compositions of the present invention are substantially free of monomeric surfactant emulsifiers. As used herein, the term "substantially free of monomeric surfactant emulsifiers" refers to a composition comprising less than about 1.0%, preferably less than about 0.5 percent, more preferably less than about 0.1 percent, and even more preferably than about 0.01 percent, or less than about 0.001 percent. By "monomeric surfactant emulsifiers", it is meant any monomeric surfactant other than those falling under the definition of "wetting agent" above. Examples of monomeric surfactant emulsifiers include non-ionic monomeric emulsifiers, such as, for example, polyoxyalkynated alcohols (alcohol alkoxylates) including mixed coconut oil derived, tallow derived, and synthetic straight-chain - primary, random, or secondary; polyoxyalkynated alkylphenols (alkylphenol alkoxylates) including polyoxyehylenated p-nonylphenol, octyl phenol, and the like.

Other examples of monomeric surfactant emulsifiers include all monomeric emulsifiers that are purely cationic such as long chain amines, diamines, and polyamines and their salts; quaternary ammonium compounds (ethoxylated or non-ehtoxylated, polyoxyethylenated long chain amines, and amine oxides, as well as, amphoteric emulsifiers, i.e., those capable of adopting a zwitterionic state (molecule having both cationic and anionic charges). Examples of these include alkylaminopropionic acids, alkyliminopropionic acids, alkylamphoacetates, alkylamphoglycinates, imidazoline carboxylates, phosphorylated imidazolines, alkylbetaines, alkylamidobetaines, certain amine oxides; sulfobetaines, and alkylsultaines, and alkyl amidosultaines.

The compositions of the present invention may further comprise any of a variety of additives or other materials used conventionally. For example, the present compositions may also include dyes, fragrances, and other functional ingredients common to skin care compositions, as long as they do not detract from the phase stability of the personal care composition. In general, in order to maintain phase stability, the level of electrolyte (e.g., ionized moieties other than the wetting agent) is maintained or substantially maintained at a relatively low level of less than 2%, such as less than about 0.5% of the total composition.

The pH of the present compositions is not critical, but may be in a range that does not facilitate irritation to the skin, such as from about 5.5 to about 7.0. The pH may be selected such that any moieties that may be present on the wetting agent that are capable of adopting a positive charge (i.e., if the wetting agent is amphoteric), do not, in fact, adopt any appreciable amount of positive charge.

The present compositions are preferably formulated to be oil-in-water emulsions that are shelf-stable in that the emulsion does not lose phase stability or "break" when kept at standard conditions (22 degrees Celsius, 50% relative humidity) for a week or more after it is made.

The viscosity of the personal care composition is not critical, although may be a spreadable cream or lotion having a viscosity greater than about 5000 centipoise when measured with a Brookfield Viscometer with an LVT2 spindle at 12 RPM.

Applicants have recognized that the compositions of the present invention may be used advantageously in a wide variety of applications. For example, in certain preferred embodiments, the present compositions are formulated to be, or be used in, personal care compositions and/or products such as, for example, diaper rash compositions, moisturizers, sunscreens, make-up or make-removal compositions, as well as other skin care compositions, and the like.

A variety of diaper rash compositions may be produced according to the present invention. Such compositions preferably comprise a hydrophilic particulate that exhibits the ability to mitigate dermatitis when applied to irritated skin. For example, in certain preferred embodiments, the diaper rash compositions of the present invention comprise zinc oxide.

Applicants have recognized that the beneficial rinse-resistance and particulate delivery properties of the present compositions allow for efficient and effective treatment of dermatitis when applied to irritated skin. Accordingly, the present composition can be used in methods of mitigating dermatitis comprising the step of applying a composition of the present invention to mammalian skin to mitigate dermatitis. The applying step may comprise applying a composition of the present invention to irritated skin covering the groin, lower stomach, upper thighs and/or buttocks of a mammal, such as a baby or infant, to mitigate redness and irritation caused, for example, by diaper dermatitis or "diaper rash."

In another embodiment of the invention, the personal care composition is applied to the skin as a moisturizer to reduce the transmission of water vapor therefrom. Suitable moisturizing compositions need not comprise a hydrophilic particulate.

In yet another embodiment of the invention, the personal care composition may include a sunscreen (e.g. titanium dioxide) and may be applied to the skin to protect the skin from the damaging effects of ultraviolet radiation.

In yet another embodiment of the invention, the personal care composition is applied to the face as a liquid makeup/foundation or make-up remover. In this embodiment, the personal care composition may include non-white pigments such as iron oxide and the like.

In certain embodiments, the personal care compositions of the present invention are preferably applied to the skin and left on the skin for a period of time such as between about 30 mintues and about 24 hours without rinsing with water of soap.

In another aspect, the present invention provides a personal care product comprising a composition of the present invention which a user applies to mammalian skin and/or hair to achieve high deposition, and/or effective barrier formation.

As used herein, the term "product' refers to a product in finished packaged form. In one embodiment, the package is a container such as a bottle, tube, jar, or other container made from, for example, plastic, metal, glass, combinations thereof, and the like, containing the composition. The product may further contain additional packaging such as a package insert and/or a plastic or cardboard box, or other outer packaging for storing such container.

Any conventional or other means for producing a product comprising a composition of the present invention may be used according to the present invention. In certain preferred embodiments, the product is a personal care product such as, for example, a cleansing product such as a conditioner, soap, facial cleanser, acne treatment, make-up remover, UV protection product, cleansing wipes, creams, such as diaper rash creams, gels, lotions, and the like. In light of the teachings herein and knowledge common in the art, those of skill in the art will be readily able to produce a product according to the present invention.

Any suitable means for directing a user to apply the composition of a product of the present invention to the skin, hair, or both, may be used. Examples of methods of directing a user include, but are not limited to, written, visual, or verbal statements made on the product, or in stores, magazines, newspaper, radio, television, internet, and the like as advertising and/or marketing for the product. In certain preferred embodiments, the product contains written instructions on the product directing the user to topically apply the composition to the skin and/or hair. Such instructions may be printed on the container, label insert, or on any additional packaging. Preferably, the written, visual, or verbal statements include a description of the high deposition and/or rinse-resistance associated with product composition and/or use of the product composition. In certain preferred embodiments, the product contains written description of the diaper rash inhibiting, high deposition and/or rinse-resistance properties associated with the product composition. Any description suitable to indicate the diaper rash inhibiting, high deposition and/or rinse-resistance associated with a product comprising a composition of the invention may be used.

### EXAMPLES

Five personal care compositions, Examples 2 and 4-7, consistent with embodiments of the invention described herein, were prepared. Six comparative compositions, Examples 1,3, and 8-11 were also prepared as described herein. Component amounts in this procedure are given in terms of parts by weight per 100 parts of the final personal care composition. Phase (A): 69.9 parts of water and 1.0 part glycerin along with 13.0 parts zinc oxide were homogenized until the solution is homogenous. 0.5 parts of the wetting agent were added with agitation. 0.6 parts ammonium acryloyldimethyltaurate/ VP Copolymer were slowly added with agitation until the solution is homogenous. Begin heating to 50°C. Phase (B) In a separate container add 9.0 parts of Mineral Oil and 6.0 parts white petrolatum were begin heated to 50°C with slow mixing. When the mixture was homogenous, phase B was poured into phase A with rapid agitation and heat was turned off. The emulsion was continually mixed until it was homogenous.

**TABLE 1**

| INCI Name | Ex 1 | Ex 2 | Ex 3 | Ex 4 | Ex 5 | Ex 6 | Ex 7 | Ex 8 | Ex 9 | Ex 10 | Ex 11 |
|---|---|---|---|---|---|---|---|---|---|---|---|
| Ammonium Acryloyldimet hyl- taurate/ VP Copolymer | 0.6 | 0.6 | 0.6 | 0.6 | 0.6 | 0.6 | 0.6 | 0.6 | 0.6 | 0.6 | 0.6 |
| Glycerin | 1.0 | 1.0 | 1.0 | 1.0 | 1.0 | 1.0 | 1.0 | 1.0 | 1.0 | 1.0 | 1.0 |
| Zinc Oxide | 13 | 13 | 13 | 13 | 13 | 13 | 13 | 13 | 13 | 13 | 13 |
| Mineral Oil | 9.0 | 9.0 | 9.0 | 9.0 | 9.0 | 9.0 | 9.0 | 9.0 | 9.0 | 9.0 | 9.0 |
| White Petrolatum | 6.0 | 6.0 | 6.0 | 6.0 | 6.0 | 6.0 | 6.0 | 6.0 | 6.0 | 6.0 | 6.0 |
| Polysorbate 20 | 0.5 | | | | | | | | | | |
| Sodium Cocoyl Isethionate | | 0.5 | | | | | | | | | |
| Disodium Nonoxynol-10 Sulfosuccinate | | | 0.5 | | | | | | | | |
| Dioctyl Sodium Sulfosuccinate | | | | 0.5 | | | | | | | |
| Sodium Methyl 2- Sulfolaurate | | | | | 0.5 | | | | | | |
| Disodium Lauret Sulfosuccinate | | | | | | 0.5 | | | | | |
| Sodium Lauryl Sulfoacetate | | | | | | | 0.5 | | | | |
| Cocamidoprop yl Betain | | | | | | | | 0.5 | | | |
| Sodium Coco PG-Dimonium Chloride Phosphate | | | | | | | | | 0.5 | | |
| Sodium Methyl Cocoyl Taurate | | | | | | | | | | 0.5 | |
| Trideceth-7 Carboxylic Acid | | | | | | | | | | | 0.5 |
| Water | 69. 9 | 69.9 | 69.9 | 69. 9 | 69.9 | 69.9 | 69.9 | 69.9 | 69.9 | 69.9 | 69.9 |

### Visual Whitening Test

A visual whitening test was performed on the various personal care compositions to determine if the skin care compositions formed a water-resistant barrier film. The procedure was accomplished by applying 1 gram of skin care composition and rubbing into the skin of the volar forearm for 15 seconds. The film was left to stand for 3 minutes then rinsed under running tap water, with gentle fmger rubbing, for three minutes.

If the zinc oxide did not readily wash off, an oily film with a white hue remains on the volar forearm. This result would be considered a PASS score for the Skin Whitening Test. If no oily film was left behind or the oily film left behind does not have a white hue (no zinc oxide in film), the result of the Skin Whitening Test is a FAIL. Results are shown in Table 2.

**TABLE 2**

| **Example #** | **Surfactant INCL Name** | **Pass/Fail Whitening Test** |
|---|---|---|
| 1 (comparative) | Polysorbate 20 | Fail |
| 2 | Sodium Cocoyl Isethionate | Pass |
| 3(comparative) | Disodium Nonoxynol-10 Sulfosuccinate | Fail |
| 4 | Dioctyl Sodium Sulfosuccinate | Pass |
| 5 | Disodium Laureth Sulfosuccinate | Pass |
| 6 | Sodium Methyl 2-Sulfolaurate | Pass |
| 7 | Sodium Lauryl Sulfoacetate | Pass |
| 8 (comparative) | Cocamidopropyl Betaine | Fail |
| 9 (comparative) | Sodium Coco PG-Dimonium Chloride Phosphate | Fail |
| 10(comparative) | Sodium Methyl Cocoyl Taurate | Fail |
| 11(comparative) | Trideceth-7 Carboxylic Acid | Fail |

Such visual whitening test may be applied to emulsions of the present invention comprising particulate hues other than white by analogy.

### Skin Whitening Test

### Methodology

Eight test sites of 2.54 cm (one inch) diameter circles were marked on the volar forearms of each subject (four test sites per arm). For each test site, a baseline reading was measured using a spectrophotometer model DTP 22 by X- Rite Inc, by placing the device on the center of the test site and measuring the Lightness (L) of the site. Once a baseline for a test site was determined, a .030mls of sample of the test material was placed on the test site. The sample was then rubbed in carefully within the one inch marked site using the subjects index finger for 20 seconds, then allowed to stand on the test site for 30 seconds. The test site was then placed under a laboratory faucet (T&S Brass Inc. Rigid gooseneck spout model B 0535 with spray head outlet model BL 5550-02) that is set to deliver 2.7 liters per minute and rinsed for 10 seconds (450 ml of water rinsed over test site). After the 10 second rinse, the area is dried with forced house air for 15-20 seconds, then air dried for two minutes. Once dried, a Lightness measurement was then taken by placing the device on the center of the test site and measuring the Lightness (L) of the site. The difference between the Lightness measurement of the experimental test site minus the baseline measurement was determined as Δ L. The technique was repeated for all eight Examples on a total of five subjects. ANOVA statistical analysis was performed on the data at a 95% Confidence Interval and results tabulated below in Table 3.

**TABLE 3**

| **Example #** | **Surfactant INCL Name** | **ΔL Avg ±SD** |
|---|---|---|
| 1 | Polysorbate 20 | -0.55 ± 1.55 |
| 2 | Sodium Cocoyl Isethionate | 3.25± 1.55 * |
| 3 | Disodium Nonoxynol-10 Sulfosuccinate | -0.67± 0.80 |
| 4 | Dioctyl Sodium Sulfosuccinate | 4.76 ± 1.53 * |
| 5 | Disodium Laureth Sulfosuccinate | 5.31 ± 2.03 * |
| 6 | Sodium Methyl 2-Sulfolaurate | 3.47 ± 2.04 * |
| 7 | Sodium Lauryl Sulfoacetate | 2.94 ±0.56* |
| 8 | Cocamidopropyl Betaine | -0.25 ± 1.02 |
| 9 | Sodium Coco PG-Dimonium Chloride Phosphate | 0.50 ± 1.04 |
| 10 | Trideceth-7 Carboxylic Acid | -0.05 ± 0.71 |
| 11 | Sodium Methyl Cocoyl Taurate | 0.12 ± 0.79 |

| | | |
|---|---|---|
| * = Statistically significantly greater than Examples # 1, 3, 8, 9, 10 and 11 at 95% Confidence Interval. | | |

Examples #2, 4, 5, 6 and 7 in both visual and instrumented skin whitening procedures have been shown to deposit significantly higher levels of zinc oxides than other wetting agents.

## Claims

1. A personal care composition, comprising:
a) a polymeric emulsifier;
b) a hydrophobic agent;
c) a wetting agent present in a weight percentage from 0.01 to 5 and selected from the group comprising disodium laureth sulfosuccinate, dioctyl sodium sulfosuccinate, sodium methyl 2-sulfolaurate, sodium cocoyl isethionate and sodium lauryl sulfoacetate; and
d) a hydrophilic particulate material which is an oxide;
wherein the composition is an oil-in-water emulsion and has a level of electrolyte, other than the wetting agent, of less than 2%.

2. The composition of claim 1, wherein the polymeric emulsifier is present in a weight percentage from 0.3 to 3.

3. The composition of claim 1, wherein the hydrophobic agent is present in a weight percentage from 0.1 to 40.

4. The composition of claim 1, wherein the hydrophilic particulate is present in a weight percentage from 0.01 to 50.

5. The composition of claim 1, wherein the polymeric emulsifier is selected from a group consisting of a hydrophobically-modified poly acrylic acid, acrylamidoalkyl sulfonic acid, cyclic N-vinylcarboxamides, and combinations thereof.

6. The composition of claim 1, wherein the hydrophobic agent is selected from a group consisting of mineral oils, vegetable oils, waxes, silicone oils, and silicone gums.

7. The composition of claim 1, wherein the hydrophilic particulate is an oxide of zinc or titanium.

8. The composition of claim 1, wherein the polymeric emulsifier is present in a concentration by weight from 0.3% to 3%, wherein the hydrophobic agent is present in a concentration from 0.1 % to 50%, wherein the polymeric emulsifier and the hydrophobic agent are present in a weight ratio from 60:1 to 1:150, and wherein the hydrophilic particulate material is present in a concentration by weight from 0.01% to 50%.

9. The composition of claim 1 wherein the polymeric emulsifier is salt-sensitive.

10. A personal care composition of claim 1 or claim 8 for use in a method of mitigating diaper dermatitis comprising applying the composition to the skin in need of diaper dermatitis mitigation.

## Patentansprüche

1. Körperpflegezusammensetzung, umfassend:
a) einen polymeren Emulgator;
b) ein hydrophobes Mittel;
c) ein Netzmittel, welches in einer Menge von 0,01 bis 5 Gew.-% vorliegt und aus der aus Dinatriumlaurylsulfosuccinat, Dioctylnatriumsulfosuccinat, Natriummethyl-2-sulfolaurat, Natriumcocoylisethionat und Natriumlaurylsulfoacetat bestehenden Gruppe ausgewählt ist; und
d) ein hydrophiles teilchenförmiges Material, bei dem es sich um ein Oxid handelt;
wobei es sich bei der Zusammensetzung um eine Ölin-Wasser-Emulsion handelt, bei der die Konzentration an Elektrolyt mit Ausnahme des Netzmittels unter 2% liegt.

2. Zusammensetzung nach Anspruch 1, wobei der polymere Emulgator in einer Menge von 0,3 bis 3 Gew.-% vorliegt.

3. Zusammensetzung nach Anspruch 1, wobei das hydrophobe Mittel in einer Menge von 0,1 bis 40 Gew.-% vorliegt.

4. Zusammensetzung nach Anspruch 1, wobei das hydrophile teilchenförmige Material in einer Menge von 0,01 bis 50 Gew.-% vorliegt.

5. Zusammensetzung nach Anspruch 1, wobei der polymere Emulgator ausgewählt ist aus einer Gruppe bestehend aus hydrophob modifizierter Polyacrylsäure, Acrylamidoalkylsulfonsäure, cyclischen N-Vinylcarboxamiden und Kombinationen davon.

6. Zusammensetzung nach Anspruch 1, wobei das hydrophobe Mittel aus einer aus Mineralölen, Pflanzenölen, Wachsen, Silikonölen und Silikongummis bestehenden Gruppe ausgewählt ist.

7. Zusammensetzung nach Anspruch 1, wobei es sich bei dem hydrophilen teilchenförmigen Material um ein Oxid von Zink oder Titan handelt.

8. Zusammensetzung nach Anspruch 1, wobei der polymere Emulgator in einer Konzentration von 0,3 Gew.-% bis 3 Gew.-% vorliegt, wobei das hydrophobe Mittel in einer Konzentration von 0,1 Gew.-% bis 50 Gew.-% vorliegt, wobei der polymere Emulgator und das hydrophobe Mittel in einem Gewichtsverhältnis von 60:1 bis 1:150 vorliegen und wobei das hydrophile teilchenförmige Material in einer Konzentration von 0,01 Gew.-% bis 50 Gew.-% vorliegt.

9. Zusammensetzung nach Anspruch 1, wobei der polymere Emulgator salzempfindlich ist.

10. Körperpflegezusammensetzung nach Anspruch 1 oder 8, zur Verwendung bei einem Verfahren zur Linderung von Windeldermatitis, bei dem man die Zusammensetzung auf die einer Linderung von Windeldermatitis bedürftige Haut aufbringt.

## Revendications

1. Composition de soin personnel, comprenant :
a) un émulsifiant polymère ;
b) un agent hydrophobe ;
c) un agent mouillant présent selon un pourcentage pondéral allant de 0,01 à 5 et choisi dans le groupe constitué par le laureth sulfosuccinate disodique, le dioctyl sulfosuccinate de sodium, le méthyl 2-sulfolaurate de sodium, le cocoyl iséthionate de sodium et le lauryl sulfoacétate de sodium ; et
d) un matériau particulaire hydrophile qui est un oxyde ;
**caractérisée en ce que** la composition est une émulsion huile-dans-eau et possède un taux en électrolytes, autres que l'agent mouillant, inférieur à 2%.

2. Composition selon la revendication 1, **caractérisée en ce que** l'émulsifiant polymère est présent selon un pourcentage pondéral allant de 0,3 à 3.

3. Composition selon la revendication 1, **caractérisée en ce que** l'agent hydrophobe est présent selon un pourcentage pondéral allant de 0,1 à 40.

4. Composition selon la revendication 1, **caractérisée en ce que** le matériau particulaire hydrophile est présent selon un pourcentage pondéral allant de 0,01 à 50.

5. Composition selon la revendication 1, **caractérisée en ce que** l'émulsifiant polymère est choisi dans le groupe constitué par un acide polyacrylique modifié de manière hydrophobe, un acide acrylamidoalkylsulfonique, des N-vinylcarboxamides cycliques, et des combinaisons de ceux-ci.

6. Composition selon la revendication 1, **caractérisée en ce que** l'agent hydrophobe est choisi dans le groupe constitué par les huiles minérales, les huiles végétales, les cires, les huiles de silicone, et les gommes de silicone.

7. Composition selon la revendication 1, **caractérisée en ce que** le matériau particulaire hydrophile est un oxyde de zinc ou de titane.

8. Composition selon la revendication 1, **caractérisée en ce que** l'émulsifiant polymère est présent selon une concentration en poids allant de 0,3% à 3%, l'agent hydrophobe est présent selon une concentration allant de 0,1% à 50%, l'émulsifiant polymère et l'agent hydrophobe sont présents selon un rapport pondéral allant de 60:1 à 1:150, et le matériau particulaire hydrophile est présent selon une concentration en poids allant de 0,01% à 50%.

9. Composition selon la revendication 1, **caractérisée en ce que** l'émulsifiant polymère est sensible aux sels.

10. Composition de soin personnel selon la revendication 1 ou la revendication 8, pour une utilisation dans une méthode d'atténuation de l'érythème fessier du nourrisson, comprenant l'application de la composition à la peau ayant besoin d'une atténuation de l'érythème fessier du nourrisson.
